# EUROPEAN PATENT APPLICATION

(11) **EP 4 667 031 A1**
(43) Date of publication of application: **24.12.2025**
(21) Application number: 24757275.3
(22) Date of filing: 15.02.2024
(51) Int. Cl.: A61M 5/20, A61M 5/315, A61M 5/32

(54) **AUTOMATIC DRUG INJECTION DEVICE**

(30) Priority: 15.02.2023 KR 20230020358; 04.04.2023 KR 20230043936
(71) Applicant: BSL CO.,LTD., Hwaseong-si Gyeonggi-do 18487 (KR)
(72) Inventor: KWON, Su Il, Anyang-si Gyeonggi-do 14102 (KR); PARK, Sung Jun, Anyang-si Gyeonggi-do 14106 (KR); LEE, Dong Ho, Gwangmyeong-si Gyeonggi-do 14315 (KR); KIM, Sung Wook, Anyang-si Gyeonggi-do 13951 (KR); HWANG, In Ho, Anyang-si Gyeonggi-do 14003 (KR)
(74) Representative: Müller Hoffmann & Partner
(86) International application number: PCT/KR2024/095201
(87) International publication number: WO 2024/172555

(57) **Abstract**

Disclosed is an auto-injector including: a tubular housing forming an exterior; a medicinal substance container including a medicinal substance storage unit storing a medicinal substance and an injection needle infiltrating a skin and injecting the medicinal substance stored in the medicinal substance storage unit and fixed to the tubular housing; an activation member slidably installed along the tubular housing, the activation member having a proximal end portion protruding outside the housing in an initial position; a first elastic member pressing the activation member in a proximal direction; a plunger including a plunger rod that slides into the medicinal substance container and presses the medicinal substance, a trigger arm extending from the plunger rod to a distal end portion, and a trigger protrusion formed to protrude outward from the distal end portion of the trigger arm; a trigger housing surrounding an outer circumferential surface of the plunger; a safety pin module installed inside the tubular housing and including a safety pin inserted between the trigger arms to cause the trigger arm to flare outward in a radial direction to maintain locking of the trigger housing and the trigger arm; and a second elastic member installed within the trigger housing and pressing the plunger in the proximal direction, wherein, as the activation member pushes the safety pin module in a distal direction, locking of the trigger arm and a locking protrusion is released and the plunger is activated.

## Description

### [Technical Field]

The present disclosure relates to an auto-injector.

### [Background Art]

In the case of chronic diseases that require continuous management, such as diabetes, or acute attacks that require immediate response, such as anaphylactic shock in allergy patients, self-injectors that allow users to inject an injectable solution on their own without visiting a hospital have been widely used.

Pen needle-type auto-injectors, which may be carried around and inserted into an injection location that meets the conditions so that a needle may be automatically inserted into the muscle and an injectable solution is automatically injected by a device, such as a spring, have been developed and widely used.

A hazardous agent injection system is disclosed in Korea Application Publication No. 10-2012-0028294.

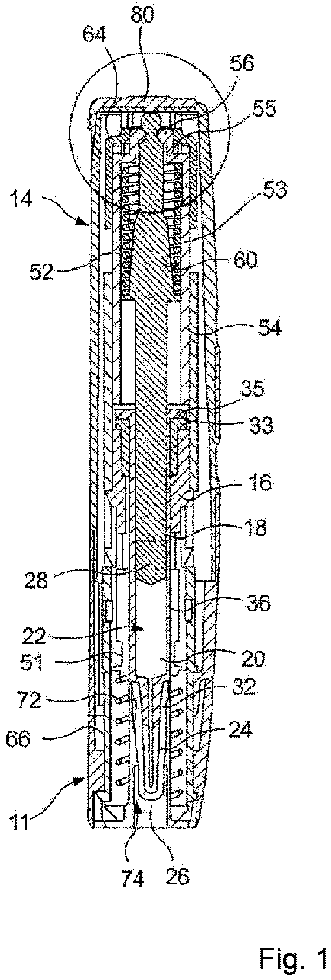

According to the related art, an injector 12 shown in FIG. 1 has an external housing member 14 configured to allow a user to handle the injector 12 and to substantially accommodate most of the illustrated components. In some embodiments, the external housing 14 is formed from two matching portions (not shown) that may be configured to be combined with each other by snap or press assembly or using adhesives, welding, etc. The housing 14 includes a fluid chamber 22 configured to store and dispense one or more liquid medicinal substances, such as one or more hazardous medical agents. The fluid chamber 22 is formed from a pre-filled syringe 18 assembled within the housing 14, but other types of fluid chambers, including cartridges of known types that may be pre-filled or refilled, or medicinal substance(s) may be used. In addition, the fluid chamber 22 may be formed integrally within the housing 14.

A locking device 80 is disposed at a distal end portion of the external housing 14, i.e. at a tip, an upper end of the housing, which is removably attached by a plurality of tabs extending through a mating opening formed on the external housing 14 to form a pressing assembly between the locking device 80 and the external housing 14. The locking device 80 is configured to prevent or reduce the possibility of unintentional discharge of the injection device while the injector 12 is being transported or handled. The locking device 80 may be removed by a user of injector 12 to allow unrestricted use of the injector 12.

In addition, a safety cap 110 may be secured to the end of the injector 12 to cover a needle guard 66 and prevent an accidental movement thereof during transport or handling prior to injection. The cap may be fixed to the end of the external housing 14 by press coupling, screw coupling, etc.

However, the auto-injector of the related art includes the locking device 80 and the safety cap 11 separately provided to prevent unintentional operation during handling, and in this case, the procedure for operating the auto injector includes three operations: removing the locking device 80, removing the safety cap 11 removal, and injecting medicinal substance.

Therefore, there is a need for the development of an auto-injector having a simplified structure to reduce the number of parts and facilitate assembling and capable of quickly injecting medicinal substances by reducing the number of procedures a user has to perform when using it.

### [Disclosure]

### [Technical Problem]

An object of the present disclosure is to provide an auto-injector capable of simplifying a structure to reduce the number of parts and facilitate assembling.

### [Technical Solution]

According to an aspect of the present disclosure, there is provided an auto-injector including: a tubular housing forming an exterior; a medicinal substance container including a medicinal substance storage unit storing a medicinal substance and an injection needle infiltrating a skin and injecting the medicinal substance stored in the medicinal substance storage unit and fixed to the tubular housing; an activation member slidably installed along the tubular housing, the activation member having a proximal end portion protruding outside the housing in an initial position; a first elastic member pressing the activation member in a proximal direction; a plunger including a plunger rod that slides into the medicinal substance container and presses the medicinal substance, a trigger arm extending from the plunger rod to a distal end portion, and a trigger protrusion formed to protrude outward from the distal end portion of the trigger arm; a trigger housing surrounding an outer circumferential surface of the plunger; a safety pin module installed inside the tubular housing and including a safety pin inserted between the trigger arms to flare the trigger arm outward in a radial direction to maintain locking of the trigger housing and the trigger arm; and a second elastic member installed within the trigger housing and pressing the plunger in the proximal direction, wherein, as the activation member pushes the safety pin module in a distal direction, locking of the trigger arm and a locking protrusion is released and the plunger is activated.

According to another aspect of the embodiment, the trigger housing may have an annular locking protrusion extending inwardly in the radial direction, and the trigger arm may flare outward in the radial direction by the safety pin and the protrusion is locked with the locking protrusion.

According to another aspect of the embodiment, the auto-injector may further include a fixing element that moves in the distal direction to fix the safety pin module removed from the interior of the trigger arm.

According to another aspect of the embodiment, the tubular housing may further include a distal cap coupled to a distal end portion, and the fixing element is formed on the distal cap.

### [Advantageous effect]

In addition, the auto-injector provided by the embodiment has the advantage of having a small number of parts and a simple structure, making it easy to assemble and simple to use.

### [Description of Drawings]

FIG. 1 is a diagram illustrating an injector equipped with an automatic feedback mechanism according to the related art;
FIG. 2 is a cross-sectional view illustrating am initial appearance of an auto-injector according to an embodiment;
FIG. 3 is a perspective view illustrating a trigger module of an auto-injector according to an embodiment;
FIG. 4 is a cross-sectional view illustrating an initial state of the trigger module of the auto-injector according to an embodiment;
FIG. 5 is a cross-sectional view illustrating a state of an auto-injector upon completion of trigger release according to an embodiment; and
FIG. 6 is a cross-sectional view illustrating a state of the auto-injector after completion of injecting a medicinal substance according to an embodiment.

### [Best Modes]

Hereinafter, embodiments will be described in more detail with reference to the drawings. However, the concept of the present disclosure may be implemented in many different forms and should not be limited to the embodiments disclosed herein, but rather, the embodiments are provided as an example intended to be thorough and complete and sufficiently convey the scope of concept to those skilled in the art.

As used herein, the term "proximal end portion" refers to an end portion of a medicinal substance transfer device in which medicinal substance release may be provided. Therefore, it is an end portion of the medicinal substance transfer device that is pointed toward an injection or release site. This definition also extends to any internal or external component of the medicinal substance transfer device, and, that is, the proximal end portion of any component is an end portion closest to the proximal end portion of the medicinal substance transfer device. A "distal end portion" is an end portion opposite to the proximal end portion.

"Proximal direction" refers to a direction from the distal end portion to the proximal end portion along the central axis of the medicinal substance transfer device. "Distal direction" refers to a direction opposite to the "proximal direction".

In addition, the term "tube type" refers to having an overall tube-like or cylindrical shape and includes slits, holes, recesses, incisions, etc. formed in a portion of the tube or cylinder, or projections, protrusions, and flange shapes.

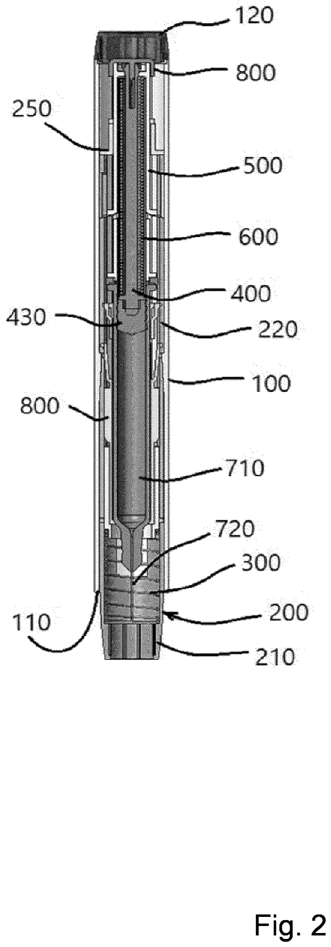

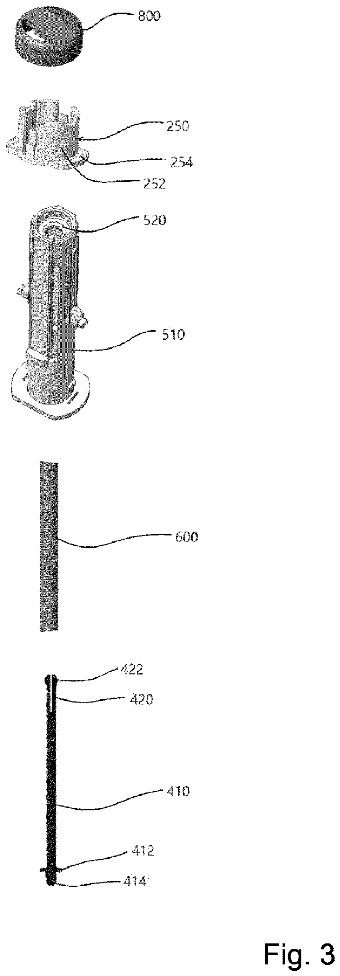

FIG. 2 is a cross-sectional view illustrating an initial state of an auto-injector according to an embodiment, and FIG. 3 is a perspective view illustrating a trigger module of the auto-injector according to an embodiment.

The auto-injector according to the embodiment includes a medicinal substance container including a medicinal substance storage unit 710 that stores a medicinal substance and an injection needle 720 that may infiltrate a skin and injects the medicinal substance stored in the medicinal substance storage unit, the medicinal substance container being fixed in a tubular housing 100 forming an exterior by the medicinal substance holder 730. Within the tubular housing 100, an activation member 200 and a first elastic member 300 pressing the activation member 200 in a proximal direction are installed on the proximal side of the medicinal substance container, and a plunger 400 pressing the medicinal substance and trigger modules 420, 500, 600, and 800 that deactivate the plunger 400 and activate the plunger 400 according to movement of the activation member 200 are installed on a distal side of the medicinal substance container.

The auto-injector according to a first embodiment includes a tubular housing 100 forming an exterior, and the tubular housing 100 includes an activation member 200 installed to slide in an axial direction therein and disposed to be adjacent to a proximal end portion of the tubular housing 100 and a first elastic member 300 that presses the activation member 200 in the proximal direction. A distal end portion 120 of the tubular housing 100 is closed and a proximal end portion 110 is open, and a proximal end portion 210 of the activation member 200 is exposed to the outside of the proximal end portion of the tubular housing 100 before a medicinal substance is injected.

Meanwhile, in an initial state, the plunger 400 is locked and deactivated by the trigger modules 420, 500, 600, and 700, and when triggering of the trigger modules 420, 500, 600, and 700 is released by movement of the activation member 200, the plunger 400 is activated and slides within the medicinal substance storage unit 710 to press the medicinal substance.

The trigger modules 420, 500, 600, and 800 are installed near the distal end portion 120 within the tubular housing 100, and the trigger module includes a trigger housing 500, a plunger 400 slidably installed in the trigger housing 500, a second elastic member 600 compressed between the trigger housing 500 and the plunger 400, and a safety pin module 800 that maintains the plunger 400 in a locked state in the trigger housing 500.

A proximal end portion of the second elastic member 600 is supported by the plunger 400, a distal end portion of the second elastic member 600 is supported by the trigger housing 500, and the second elastic member 600 is installed in a compressed state to press the plunger 400 in the proximal direction.

Until the activation member 200 slides in the distal direction to release the trigger of the trigger module, the plunger 400 is locked by the trigger modules 420, 500, 600, and 800 not to move in the proximal direction even when the pressure of the second elastic member 600 is applied.

Until the activation member 200 slides in the distal direction to release the trigger of the trigger module, the plunger 400 is locked not to move in the proximal direction even when the pressure of the second elastic member 600 is applied.

The proximal end portion 210 of the activation member 200 has a tubular shape and has a contact surface, which comes into contact with a user's skin, at a tip of the proximal end portion 210. A hole is provided in the center of the contact surface, and when the activation member 200 retracts, an injection needle 720 is exposed through the hole.

The activation member 200 further includes an activation arm 220 extending in the distal direction from proximal end portion 210. The activation arm 220, while moving in the distal direction, pushes the safety pin module 800 in the distal direction to unlock the trigger modules 420, 500, 600, and 800 and activates the plunger 400. In an embodiment, an activation sleeve 250 is further provided in the distal direction of the activation member 200, the activation arm 220 pushes the activation sleeve 250, and the activation sleeve 250 pushes the safety pin module 800. However, the activation sleeve 250 may be omitted and the activation arm 220 may directly push the safety pin module 800.

The plunger 400 includes a plunger rod 410 that slides into the medicinal substance container 710 and injects a medicine and a plurality of trigger arms 420 extending in the distal direction from a distal end portion of the plunger rod 410. An annular support portion 412 for supporting the second elastic member 600 is formed near the proximal end portion of the plunger rod 410. The plurality of trigger arms 420 are formed at intervals from each other along an outer circumference of the distal end portion of the plunger rod 410, the trigger arms 420 are formed at intervals from each other, and the safety pin 830 of the safety pin module 800, which will be described below, is inserted into the interval. A trigger protrusion 422 protruding outward in the radial direction is formed at a distal end portion of the trigger arm 420. In addition, a packing portion 430, which is inserted into the medicinal substance storage portion 710 to be described below and packs so that the medicinal substance is injected through the injection needle 720 without leaking when the plunger 400 moves, is installed at a proximal end portion of the plunger rod 410.

The trigger housing 500 includes a tubular body 510, and an annular locking protrusion 520 is formed along an inner circumferential surface of the body 510 so as to be adjacent to a distal end portion thereof. A distal end portion of the trigger arm 420 penetrates through the trigger housing 500 through a hollow in the middle of the locking protrusion 520.

The safety pin module 800 is coupled to the distal end portion of tubular housing 100. The safety pin module 800 includes a tubular body 810, a head portion 820 formed at a distal end portion of the tubular body 810, and a safety pin 830 protruding in the proximal direction from the head portion 820 and is inserted between the trigger arms 420.

The trigger housing 500 is fixed to the tubular housing 100, and the distal end portion of the trigger housing 500 is located within the safety pin module 800, that is, within the tubular body 810.

The trigger protrusion 422 of the trigger arm 420, when the safety pin 830 is inserted, flares outward in the radial direction, and is locked with the locking protrusion 520 of the trigger housing 500. The trigger housing 500 and the trigger arm 420 are not unlocked unless the safety pin 830 of the safety pin module 800 is removed from the cavity between the trigger arms 420. At this time, the trigger arm 420 is preferably elastically movable in the radial direction, and if the safety pin 830 is not inserted into the middle of the trigger arms 420, a protruding radius of the trigger protrusion 422 is preferably smaller than a diameter of an inner circumferential surface of the locking protrusion 520 of the trigger housing. Accordingly, when the safety pin 830 is removed, the trigger arm 420 moves to its original position and the locking of the locking protrusion 520 and the trigger protrusion 422 is easily released.

When the activation member 200 moves in the distal direction from the initial position due to pressure from the user, the safety pin module 800 moves from a first position for locking the trigger module to a second position for releasing the trigger of the trigger module.

At this time, a fixing element 122 may be provided to fix the position of the safety pin module 800 in a state in which the safety pin module 800 has moved to the second position. In an embodiment illustrated in FIGS. 2 and 3, the tubular housing 100 has a distal cap 120 that closes the distal end portion, and the distal cap 120 includes the fixing element 122 that fixes the safety pin module 800 in the second position. The fixing element 122 has a hook shape protruding inward, and the fixing element 122 fixes the proximal end portion of the tubular body 810 as the safety pin module 800 moves to the second position. Thereafter, even if the activation member 200 moves again in the proximal direction and returns to the initial position, the safety pin module 800 is fixed to the second position.

A medicinal substance container 700 including a medicinal substance storage unit 710 storing the medicinal substance and an injection needle 720 injecting the stored medicinal substance is installed in the tubular housing 100. The medicinal substance container 700 is maintained in a fixed position in the tubular housing 100, regardless of the movement of the activation member 200 or the plunger 400.

Here, the proximal end portion of the plunger 400 is assembled in a state of being inserted into the medicinal substance storage unit 710 at a predetermined depth, so that when the second elastic member 600 presses the plunger 400, the plunger 400 moves in the proximal direction within the medicinal substance storage unit 710, while the medicinal substance is injected into the user's body through the injection needle 720.

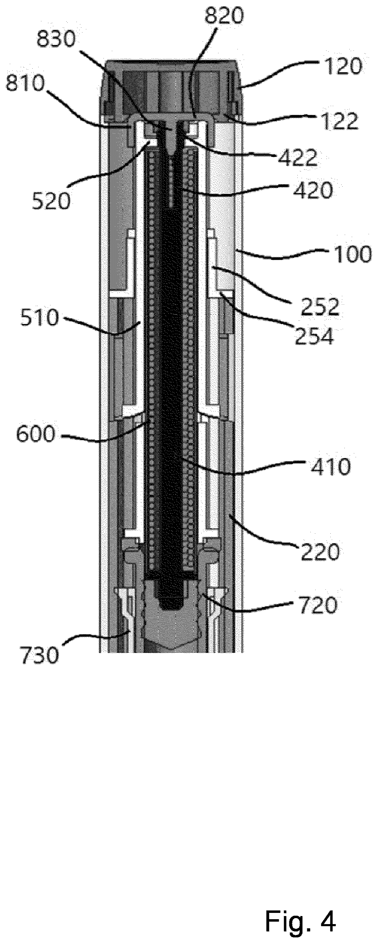

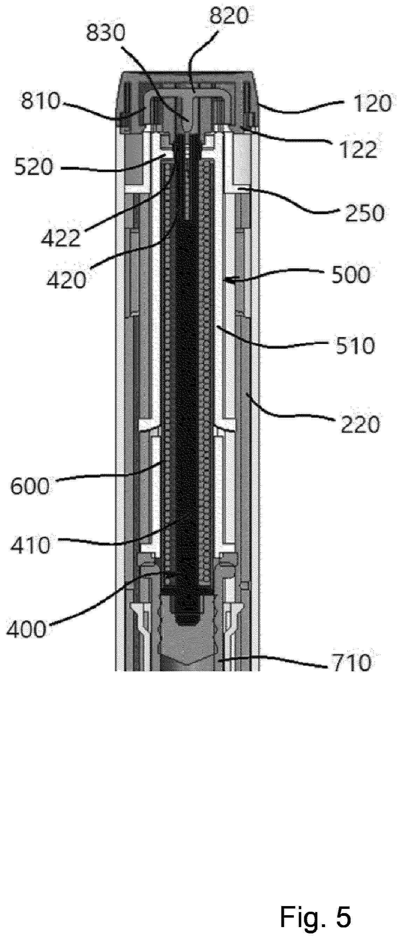

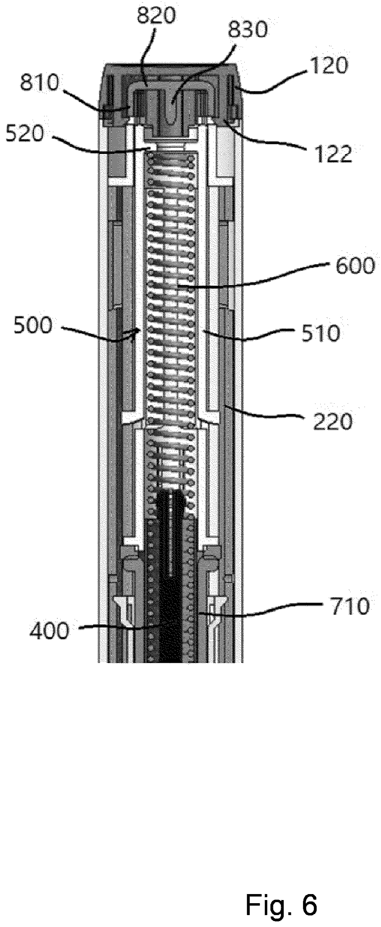

FIG. 4 is a cross-sectional view illustrating an initial state of the trigger module of the auto-injector according to an embodiment, FIG. 5 is a cross-sectional view illustrating a state of an auto-injector upon completion of trigger release according to an embodiment, and FIG. 6 is a cross-sectional view illustrating a state of the auto-injector after completion of injecting a medicinal substance according to an embodiment.

As described above, when the activation member 200 moves in the distal direction, the activation arm 220 pushes the head portion 810 of the safety pin module 800 in the distal direction, thereby releasing the safety pin 830 and releasing trigger of the trigger module. The safety pin 830 is removed, and the trigger protrusion 422 of the trigger arm 420 moves radially inward, thereby easily unlocking between the trigger protrusion 422 and the locking protrusion 520 of the trigger housing 500.

When the locking is released, the plunger 400 receives pressing force in the proximal direction due to restoring force of the compressed second elastic member 600, and accordingly, the plunger 400 moves in the proximal direction within the medicinal substance storage unit 710 to press the medicine such that the medicine is injected into the user's body through a needle.

## Claims

1. An auto-injector comprising:
a tubular housing forming an exterior;
a medicinal substance container including a medicinal substance storage unit storing a medicinal substance and an injection needle infiltrating a skin and injecting the medicinal substance stored in the medicinal substance storage unit and fixed to the tubular housing;
an activation member slidably installed along the tubular housing, the activation member having a proximal end portion protruding outside the housing in an initial position;
a first elastic member pressing the activation member in a proximal direction;
a plunger including a plunger rod that slides into the medicinal substance container and presses the medicinal substance, a trigger arm extending from the plunger rod to a distal end portion, and a trigger protrusion formed to protrude outward from the distal end portion of the trigger arm;
a trigger housing surrounding an outer circumferential surface of the plunger;
a safety pin module installed inside the tubular housing and including a safety pin inserted between the trigger arms to cause the trigger arm to flare outward in a radial direction to maintain locking of the trigger housing and the trigger arm; and
a second elastic member installed within the trigger housing and pressing the plunger in the proximal direction,
wherein, as the activation member pushes the safety pin module in a distal direction, locking of the trigger arm and a locking protrusion is released and the plunger is activated.

2. The auto-injector of claim 1, wherein
the trigger housing has an annular locking protrusion extending inwardly in the radial direction, and
the trigger arm flares outward in the radial direction by the safety pin and the protrusion is locked with the locking protrusion.

3. The auto-injector of claim 1, further comprising a fixing element that moves in the distal direction to fix the safety pin module removed from the interior of the trigger arm.

4. The auto-injector of claim 3, wherein the tubular housing further includes a distal cap coupled to a distal end portion, and the fixing element is formed on the distal cap.
